# EUROPEAN PATENT SPECIFICATION

(11) **EP 1 980 216 B1**
(45) Date of publication and mention of the grant of the patent: **02.02.2011**
(21) Application number: 08010167.8
(22) Date of filing: 10.12.2004
(51) Int. Cl.: A61B 17/32, A61B 17/16

(54) **Articular cartilage repair implant delivery device**
Vorrichtung zum Einsetzen eines Implantats für Gelenkknorpelreparatur
Dispositif de mise en place d'implants de réparation de cartilage articulaire

(30) Priority: 23.12.2003 US 746749
(43) Date of publication of application: 15.10.2008
(62) Divisional of application: 04257681.9
(73) Proprietor: DePuy Products, Inc., Warsaw, IN 46581 (US)
(72) Inventor: Jacobs, Andrew M., Fort Wayne IN 46804 (US); Zannis, Anthony D., Fort Wayne IN 46804 (US); Schwartz, Herbert E., Fort Wayne IN 46845 (US); Day, Carolyn K., Maumee OH 43537 (US); Kempainen, John, Richland MI 49083 (US)
(74) Representative: Belcher, Simon James

(56) References cited:
- WO-A-96/24304
- WO-A-03/051210
- US-A- 4 895 148

## Description

The present invention relates to repair of articular cartilage in joints and, more particularly, to instruments for preparing subchondral tissue and bone, and implanting an articular cartilage repair unit therein.

Articular cartilage is a type of hyaline cartilage that lines the surfaces of the opposing bones in a diarthrodial joint (e.g., knee, hip, shoulder, etc.). Its primary function is to permit smooth, near frictionless movement during articulation between bones of the joint by providing a low-friction interface between the contacting cartilage surfaces of the joint. Articular cartilage is also load bearing, and serves to transmit and distribute compressive joint loads to the underlying subchondral bone (i.e. bone beneath the cartilage or subchondral tissue).

Articular cartilage is typically damaged in one of two ways, acute trauma inflicted through physical activity (such as twisting motion of the leg, sharp lateral motion of the knee, or repetitive impact), or degenerative conditions (such as arthritis or other systemic conditions). In addition, as a person ages, articular cartilage loses mechanical strength, rendering the cartilage even more susceptible to trauma, such that even common motions (e.g. squatting, stair climbing, etc.) can cause articular cartilage tears. Because articular cartilage tissue is aneural (little or no nerves) and avascular (little or no blood vessels), the spontaneous healing of damaged articular cartilage is limited. As a result, focal (localized) defects can tend to lead toward progressive degeneration of the joint surface(s) until total joint replacement is eventually necessary.

Surgical methods are available for treatment of damaged articular cartilage tissue with their aim being to partially or completely repair the chondral defect and decrease the risk of the development of osteoarthritic changes within the joint. These surgical methods can be loosely classified into three categories: 1) debridement and stabilization of loose or worn articular cartilage, 2) stimulation of a repair process from the subchondral bone, and 3) repair or replacement of the damaged articular surface.

With respect to debridement and stabilization of loose or worn cartilage, the basic strategy is to stabilize the defect by removal of any partially attached flap(s) of cartilage or badly worn areas that may be present. This method typically involves procedures such as arthroscopic debridement of the loose cartilage and removal of any detached cartilage tissue bodies that may be floating with the joint space, which could also be a potential source of inflammation. Arthroscopic debridement is considered when medical management has failed to satisfactorily alleviate symptoms. In addition, it is generally agreed that while this method is able to produce short-term alleviation of pain, the long-term effect is frequently eventual deterioration of the joint surface(s).

With respect to stimulation of a repair process from the subchondral bone, the basic strategy is to enhance the intrinsic capacity of the cartilage (and the subchondral bone) to heal itself. This is done by stimulating healing by recruiting cells from the underlying bone marrow in the subchondral bone. This method typically involves penetrating the subchondral bone by drilling, microfracture, or abrasion. It is generally agreed that with the techniques that are available today for executing this method, the results are similar to debridement and stabilization in that short-term pain may be alleviated through the growth of fibrous tissue. However, over the long-term, the effect typically is the eventual deterioration of the joint surface(s).

With respect to repair or replacement of the damaged articular surface, the basic strategy is to regenerate a new joint surface by transplanting chondrocytes, chondrogenic cells or tissue that has the potential to grow new cartilage. This method typically involves techniques such as osteochondral autographing (mosaicplasty), in which "plugs" of cartilage tissue and subchondral bone are harvested from a patient and implanted into the damaged cartilage area, or autologous chondrocytes transplantation, in which cartilage cells are harvested from a patient, cultured, and then implanted into the damaged cartilage area.

While the most preferred treatment method is restoration and repair of the damaged cartilage, the most common method today is debridement and stabilization, followed by stimulation of a repair process from the subchondral bone. Additionally, and as indicated above, while the first two categories of methods may be effective in the short-term relief of pain and discomfort to the patient, the long-term effect is typically an eventual deterioration of the cartilage surface, leading to the need for a total joint replacement.

With respect to repair of damaged articular cartilage, various devices have been developed in order to facilitate such repair. A device has been developed by DePuy Orthopaedics Inc which is referred to as an Articular Cartilage Repair Unit (ACRU), and whose function is based on the second category explained above, i.e. stimulation of a repair process from the subchondral bone. The ACRU utilizes a resorbable mesh material that acts as a scaffold or matrix for cell infiltration and proliferation.

The ACRU and other implants requires that the material be removed from the existing defect to expose the subchondral bone, allowing the scaffold or matrix and autologous cells access to stimulate the healing of the tissue. Such implants are preferably implanted arthroscopically. Particularly, the general surgical technique for implantation of the implant requires that the articular cartilage surrounding the damaged cartilage be prepared. Thereafter, the subchondral bone beneath the damage cartilage is prepared, and a pilot hole prepared into the subchondral bone.

Although various techniques and instruments have been developed that have attempted to address the need for articular cartilage repair, such techniques and instruments have not been optimal. For instance, either they are not arthroscopic in nature (as disclosed in US-6171340) or have a relatively difficult surgical procedure associated with such implantation (as disclosed in WO-96/24304 and WO 03/051210).

The present invention provides improved devices, especially arthroscopic device, for preparing an articular cartilage site for implantation of an articular cartilage repair device, and for implanting an articular cartilage repair device into subchondral bone.

Accordingly, in one aspect, the present invention provides a set of surgical tools for preparing a damaged articular cartilage site on subchondral bone of a joint and implanting an articular cartilage repair device, as defined in claim 1.

A technique for the arthroscopic delivery and fixation of an articular cartilage repair unit (ACRU) fixation device or implant is provided. The technique includes the use of a cannula tube that functions as both a cartilage cutter and a guide to pass instruments into the body arthroscopically. One such instrument is an end-cutting reamer that both prepares the subchondral bone by re-surfacing it down to a specified depth and also simultaneously drills a pilot hole in the subchondral bone to accept the ACRU fixation device. A delivery device is utilized to hold and deliver the ACRU fixation device to the delivery site.

The instrument of the invention includes a combination articular cartilage cutter and instrument guide for preparation of an articular cartilage site and/or the implantation of an articular cartilage repair device in subchondral bone.

The instrument of the invention includes a combination articular cartilage reamer and implantation borer for implantation of an articular cartilage repair device into subchondral bone.

The cannula includes a tubular body that defines a proximal and a distal end, a bore disposed in the tubular body and extending from the proximal end to the distal end, and a blade disposed at a tip of the distal end of the tubular body and configured to incise about an articular cartilage area.

The cannula provides a surgical instrument guide and articular cartilage cutter. The surgical instrument guide and articular cartilage cutter includes a tubular body having a longitudinal instrument bore extending from a proximal end of the tubular body to a distal end of the tubular body, a surgical instrument stop defined at the proximal end of the tubular body and configured to provide an abutment for limiting a length of travel of a surgical instrument, and an articular cartilage cutting blade defined at the distal end of the tubular body and configured to incise about an articular cartilage area.

The surgical drill includes a shaft defining a proximal end and a distal end, an attachment head disposed at the proximal end of the shaft and configured to be received in a rotation device, and a reamer disposed at the distal end of the shaft and configured to ream about an incised articular cartilage area and to simultaneously prepare a bore in subchondral bone underneath the incised articular cartilage area.

The surgical drill includes a drill shaft having a proximal end and a distal end, an attachment tip on the proximal end of the drill shaft and configured to be received in a rotation device, and a site preparation tip on the distal end of the drill shaft and configured to ream an incised area of the damage damaged articular cartilage and to simultaneously prepare a bore in the subchondral bone underneath the reamed area of the damaged articular cartilage.

The implant delivery device includes a handle defining a proximal end and a distal end, a shaft extending from the distal end of the handle and having an application end with a retention slot configured to releasably receive an articular cartilage repair assembly comprising an articular cartilage repair unit releasably retained on an articular cartilage implant retainer, and a retaining sleeve disposed on the shaft and operative in a first mode to allow the articular cartilage implant retainer to be received in the retention slot, and in a second mode that prevents egress of the articular cartilage implant retainer from the retention slot.

The implant delivery device includes a tubular sleeve having a longitudinal bore and defining a proximal end and a distal end, and an insert extendable from and retractable into the longitudinal bore of the tubular sleeve, the insert having an application end with a retention slot configured to releasably receive an articular cartilage repair assembly comprising an articular cartilage repair unit releasably retained on an articular cartilage implant retainer.

The cannula has a tubular body defining a proximal and a distal end a bore disposed in the tubular body and extending from the proximal end to the distal end, a blade disposed at a tip of the distal end of the tubular body and configured to incise about an articular cartilage area. The surgical drill has a drill shaft having a proximal end and a distal end, an attachment tip on the proximal end of the drill shaft and configured to be received in a rotation device, and a site preparation tip on the distal end of the drill shaft and configured to ream an incised area of the damaged articular cartilage and to simultaneously prepare a bore in the subchondral bone underneath the reamed area of the damaged articular cartilage. The implant delivery device has a handle defining a proximal end and a distal end, a shaft extending from the distal end of the handle and having an application end with a retention slot configured to releasably receive an articular cartilage repair assembly comprising an articular cartilage repair unit releasably retained on an articular cartilage implant retainer. The retaining sleeve is disposed on the insert and is operative in a first mode to allow the articular cartilage implant retainer to be received in the retention slot, and in a second mode that prevents egress of the articular cartilage implant retainer from the retention slot.

The components provided by the present invention can be used in a method of implanting an articular cartilage repair device into an area of damaged articular cartilage. The technique includes the steps of (a) providing access to a joint space having damaged articular cartilage through an arthroscopic portal in the body of a patient, (b) using an obturator to insert a combination instrument guide and articular cartilage cutter cannula through the arthroscopic portal, (c) removing remnants of damaged articular cartilage at a damaged articular site through the inserted cannula, (d) simultaneously preparing the damaged articular cartilage site and underlying bone with a surgical drill having a combination reaming and boring tip through the cannula, and (e) implanting an articular cartilage repair assembly onto the prepared articular cartilage site through the cannula.

The components provided by the present invention can be used in a method of implanting an articular cartilage repair device comprising the steps of:
providing access to a joint space having damaged articular cartilage through an arthroscopic portal in the body of a patient;
using an obturator to insert a combination instrument guide and articular cartilage cutter cannula through the arthroscopic portal;
removing remnants of damaged articular cartilage at a damaged articular site through the inserted cannula;
simultaneously preparing the damaged articular cartilage site and underlying bone with a surgical drill having a combination reaming and boring tip through the cannula; and
implanting an articular cartilage repair assembly onto the prepared articular cartilage site through the cannula.

The step of simultaneously preparing the damaged articular cartilage site and underlying bone with a surgical drill having a combination reaming and boring tip through the cannula can include re-surfacing the underlying subchondral bone a particular depth and drilling a pilot hole for receipt of a fixation device of the articular cartilage repair assembly.

Embodiments of the invention will now be described by way of example with reference to the accompanying drawings, in which:
Fig. 1 is a side view of one embodiment of a cannula in accordance with an aspect of the subject invention;
Fig. 2 is an enlarged side view of the distal tip of the cannula of Fig. 1 taken along circle 2-2 thereof;
Fig. 3 is a perspective view of an obturator in accordance with an aspect of the subject invention that may be used with the cannula of Figs. 1 and 2;
Fig. 4 is a side view of a reamer in accordance with an aspect of the subject invention that may be used with the cannula of Figs. 1 and 2;
Fig. 5 is a side view of another cannula in accordance with the subject invention;
Fig. 6 is a sectional view of the cannula of Fig. 5 taken along line 6-6 thereof;
Fig. 7 is a sectional view of another reamer in accordance with an aspect of the subject invention that may be used with the cannula of Figs. 5 and 6;
Fig. 8 is an enlarged sectional view of the cutting tip assembly of the reamer of Fig. 7;
Fig. 9 is an enlarged top perspective view of the cutting blade portion of the cutting tip assembly of the reamer of Fig. 7;
Fig. 10 is a fragmentary sectional view of a portion of an articular cartilage depth gauge in accordance with an aspect of the subject invention;
Fig. 11 is a fragmentary side view of a portion of the depth gauge pin illustrating the degree of curvature in accordance with an aspect of the subject invention;
Fig. 12 is fragmentary perspective view of an implant delivery device in accordance with an aspect of the subject invention, the implant delivery device shown in a pre-loaded non-extended state;
Fig. 13 is a fragmentary perspective view of the implant delivery device of Fig. 12 shown in a pre-loaded extended state ready for receipt of an ACRU assembly;
Fig. 14 is a perspective view of an ACRU implant assembly for use in the implant delivery device of Fig. 12;
Fig. 15 is a perspective view of an implant retainer for use with the implant delivery device of Fig. 12;
Fig. 16A is a bottom perspective view of an ACRU implant assembly with an implant retainer in accordance with an aspect of the subject invention;
Fig. 16B is a side view of the ACRU implant assembly with an implant retainer of Fig. 16A;
Fig. 17 is a fragmentary side view of the implant delivery device in a loaded and extended state;
Fig. 18A is an enlarged perspective view of another embodiment of an implant retainer in accordance with an aspect of the subject invention;
Fig. 18B is an enlarged perspective view of another embodiment of an implant retainer in accordance with an aspect of the subject invention;
Fig. 18C is an enlarged perspective view of another embodiment of an implant retainer in accordance with an aspect of the subject invention;
Fig. 19 is a perspective view of another implant delivery device in accordance with an aspect of the subject invention, the implant delivery device in an unloaded state;
Fig. 20 is a perspective view of the implant delivery device of Fig. 19, the implant delivery device in a pre-load state;
Fig. 21 is a perspective view of the implant device of Fig. 19, the implant delivery device in an initially loaded state;
Fig. 22 is a perspective view of the implant device of Fig. 19, the implant device in a loaded and ready to implant state;
Fig. 23 is a perspective view of yet another implant device in accordance with an aspect of the subject invention, the implant delivery device in an unloaded state;
Fig. 24 is a perspective view of the implant delivery device of Fig. 23, the implant delivery device in a pre-loaded state;
Fig. 25 is a perspective view of the implant device of Fig. 23, the implant delivery device in an initially loaded state;
Fig. 26 is a perspective view of the implant device of Fig. 23, the implant device in a loaded and ready to implant state;
Fig. 27 is a perspective view of another alternative embodiment of an implant delivery device;
Fig. 28 is a side view of the tip portion of the implant delivery device of Fig. 27 with an ACRU assembly attached thereto;
Fig. 29 is an enlarged side view of an ACRU implant assembly with implant retainer wire for purposes of illustrating the manner in which the ACRU assembly is implanted, the ACRU assembly shown in an initially implanted state;
Fig. 30 is an enlarged side sectional view of the ACRU implant assembly with implant retainer wire of Fig. 29 illustrating the beginning of ACRU assembly release;
Fig. 31 is an enlarged side sectional view of the ACRU implant assembly with implant retainer wire of Fig. 29 illustrating continuous ACRU assembly release;
Fig. 32 is an enlarged side sectional view of the ACRU implant assembly with implant retainer wire of Fig. 29 illustrating a final phase of ACRU assembly release;
Fig. 33 is a side sectional view of a portion of subchondral bone in which the ACRU implant assembly of Fig. 29 has been implanted;
Fig. 34 is a side view of an alternative embodiment of an implant delivery device;
Fig. 35 is a side view of a curved cannula in which the implant delivery device of Fig. 34 may be utilized; and
Fig. 36 is a flowchart of a method or technique utilizing the instruments of the present invention in accordance with the subject invention.

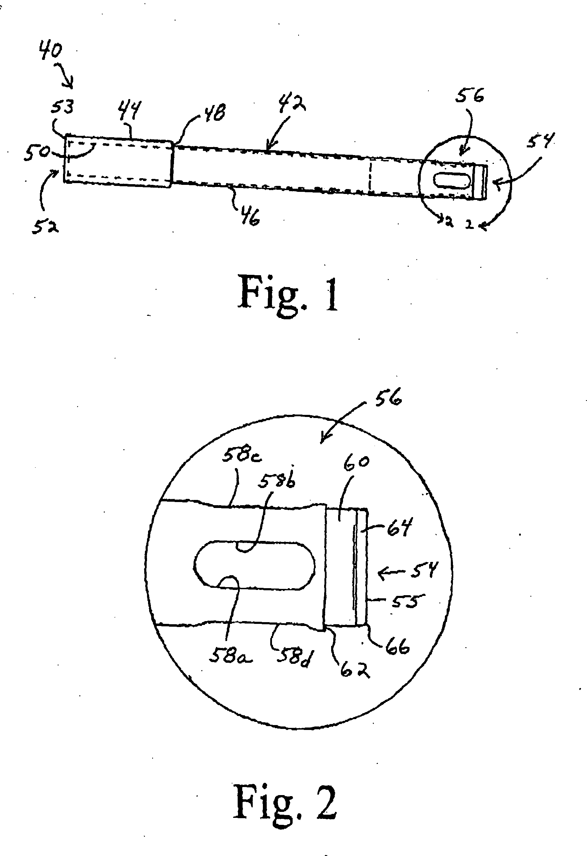

Referring to the drawings, Fig. 1 shows a cannula or cannula construct 40 in accordance with the subject invention. While the cannula 40 is an instrument which can be used independently of other instruments, the cannula 40 can be used as one of a set of instruments or devices for performing a surgical procedure on articular cartilage, the other instruments of the set being shown in the other figures and described herein. While described more fully below, the surgical procedure and one or more of the various instruments of the present instrument set, guides the various instruments through an incision of the body, gauges the depth of articular cartilage at a cartilage defect or focal lesion (site) of a joint that is proximate the incision, prepares the site, and allows implanting of an ACRU at the site.

The cannula 40 is made from a suitable surgical material such as metal and, preferably, but not necessarily, from stainless steel. Other suitable materials, however, can be used and are contemplated. The cannula 40 is defined by a cylindrical, tubular or similarly shaped body 42 having a first portion 44 and a second portion 46. A bore 50 extends the axial length of the body 42 through the first portion 44 and the second portion 46. The bore 50 defines a first opening 52 in an axial (proximal) end of the first portion 44 and a second opening 54 in an axial end (distal tip 56) of the second portion 46.

While the bore 50 may have the same diameter throughout the first portion 44 and the second portion 46, the outer diameter of the first portion 44 is greater than the outer diameter of the second portion 46. This creates an annular ledge 48 defining a boundary between the outer diameter of the first portion 44 and the outer diameter of the second portion 46. The bore 50, and thus the cannula 40 itself, serves as a guide to pass and/or utilize the various instruments of the present instrument set (and other instruments not shown and/or described herein) through an incision in the body. While the incision and the procedure is preferably arthroscopic in nature, the various instruments of the present instrument set may be utilized in surgical procedures that are not arthroscopic in nature.

It should be appreciated that the bore 50 may be made in various diameters. As such, the outer diameter of the first and second portions 44, 46 may be fashioned in various diameters. A surgical set of cannula tubes 40 of various outer diameters and/or various diameter bores may be provided. In this manner, a surgeon may have a choice of sizes of cannula 40 in order to accommodate ACRU implant assemblies of varying diameters.

Referring additionally to Fig. 2, the cannula 40 terminates at a distal end in a distal tip 56. The distal tip 56 is configured to be a cartilage cutter that is utilized to prepare and/or aid in the preparation of the articular cartilage site. The distal tip 56 includes one or more windows or openings 58. The distal tip 56 of the cannula 40 has four windows 58a, 58b (that is diametrically opposite window 58a and thus is not readily discernable in the figures)58c and 58d (that is diametrically opposite window 58c). Placement and number of windows may vary as desired and/or appropriate. Additionally, the windows 58a-d are fashioned as ovals or ovoid shaped openings. The shape and/or size of the windows may vary.

The distal tip 56 also has an annular cutting section 60. The cutting section 60 is here formed as a reduced diameter portion and as such, defines an annular rim 62. At the end of the cutting section 60 is a blade 64. The blade 64 is annular and defined by a tapered, angled or bevelled edge 66. The blade 64 is shown as annular, but may be fashioned in other shapes if desired. Typically, the blade has the same shape as a cross section of the body of the cannula. The blade 64 is adapted, configured, and/or operative to cut an area of cartilage (e.g. a focal lesion or damaged cartilage) from the articular cartilage site. The length of the blade 64 determines the maximum depth of articular cartilage cut. The incised area of cartilage corresponds to the shape of the blade 64 and thus corresponds, as well, to the size of the bore 50 at the distal bore end 54. The cannula 40 is both a guide for instruments via passing of an instrument through the bore 50 thereof, and an articular cartilage cutter through utilization of the distal tip 56. The cannula 40 is thus a dual purpose instrument.

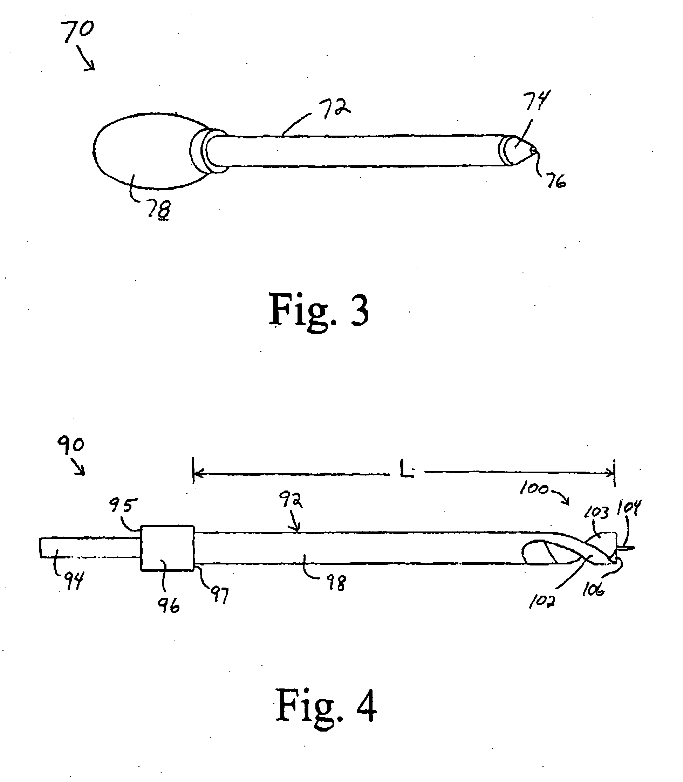

Referring now to Fig. 3, there is shown an obturator generally designated 70 fashioned in accordance with an aspect of the subject invention. As such, the obturator 70 may be part of the instrument set for preparing the articular cartilage site and implanting an ACRU. The obturator 70 is characterized by a body 72 formed as an elongated tube, cylinder or the like. The body 72 is fabricated from a material suitable for surgical use. The body 72 terminates at a distal conical shaped end 74 in a rounded tip 76.

The body 72 is of a diameter that permits the body 72 to pass through the bore 50 of the cannula 40. As well, the body 72 is of sufficient length to allow the tip 76 to either extend beyond or be at/near the end 55 (see Fig. 2) of the cannula 40 when the body 72 is passed into and through the bore 50 of the cannula 40. This places the tip 76 proximate the articular cartilage site when the cannula 40 has been inserted into the body of a patient through an appropriate (e.g. regular or arthroscopic) incision. It should be appreciated that the articular cartilage site refers to a damaged area of articular cartilage.

A handle 78 is provided at a proximal end of the body 72. The body 72 is also of sufficient length to allow the bulb 78 to be sufficiently beyond the end 53 (see Fig. 1) of the cannula 40 when the tip 76 is in a proper position with respect to the cannula 40. The obturator 70 may be fabricated in various sizes (e.g. tube length, tube outer diameter, tube or hollow inner diameter) to be accommodated in various sizes of cannula.

Referring now to Fig. 4, there is depicted a reamer generally designated 90 in accordance with an aspect of the subject invention. As such, the reamer 90 may be part of the instrument set for preparing the articular cartilage site and implanting an ACRU as described herein. The reamer 90 is characterized by a body 92 that is fabricated from a suitable surgical material such as metal (e.g. stainless steel) or other material as appropriate. The body 92 includes a shaft 94 that is configured and/or adapted to be attached to a drill (not shown) or like rotation device for rotating the reamer 90. As such, the shaft 94 may be configured in various manners in order to be accommodated in the rotation device. The attachment shaft 94 terminates in a stop or stop portion 96 that is sized larger than the attachment shaft 94. The stop portion 96 may be annular or cylindrical in shape. Since the stop portion 96 is larger (i.e. has a greater diameter than the attachment shaft 94), the stop portion 96 defines a stop surface 95 at the junction of the attachment shaft 94 and the stop portion 96. The stop surface 95 provides a stop for insertion of the reamer 90 into the rotation device.

The body 92 further includes an elongated shaft 98 that is connected to the opposite side of the stop portion 96. The shaft 98 is preferably cylindrical and has a diameter that is able to be accommodated in the bore 50 of the cannula 40. The diameter of the elongated shaft 98, however, is less than the diameter of the stop portion 96. As such, a stop surface 97 is defined at the junction of the stop portion 96 and the elongated shaft 98. The stop surface 97 provides a stop for insertion of the reamer 90 in the cannula 40. Particularly, when the reamer 90 is fully inserted into the bore 50 of the cannula 40, the stop surface 97 abuts the end 53 of the first portion 44 of the body 42 of the cannula 40.

The reamer 90 includes a cutting or reaming head or portion 100 at the distal end of the elongated shaft 92 that is adapted, configured and/or operative to cut through cartilage (e.g. meniscus and articular) and to simultaneously bore a hole in subchondral bone. As such, the reaming head 100 has helical blades 102 and 103. Of course, it should be appreciated that while two helical blades are shown, the reaming head 100 may have more helical blades and/or other styles of blades.

A cutting tip 104 axially extends from an end 106 of the blades 102, 103. The cutting tip 104 is adapted, configured and/or operative to bore a hole in the subchondral bone underneath the reamed articular cartilage. The size and length of the cutting tip 104 is selected so as to bore a hole appropriate for receiving the stem of an articular repair device such as is known in the art. Thus, as the reamer 90 is rotated, the appropriate sized hole is bored into the subchondral bone while the blades 102, 103 ream out the previously incised area of articular cartilage.

The reamer 90 (i.e. the various parts thereof) may be fashioned in various dimensions in order to be accommodated in a particular cannula and/or to effect appropriate cutting depths of both the bore in the subchondral bone for the shaft of the articular cartilage repair device and the cartilage at the cartilage repair site (having been previously cut via the cutting blade 66 of the cutting head 56 of the cannula 40). In all cases, however, the length L of the elongated shaft portion 98 including the cutting portion 100 (i.e. from the stop surface 97 to the end 106) is sized such that when the stop surface 97 abuts the end 53 of the cannula 40, a portion of the blades 102, 103 (from the end 106 rearward toward the stop surface 97) extends beyond the end 55 of the cutting portion 56 of the cannula 40. The amount of blade extension is sufficient to ream out the incised depth of the articular cartilage.

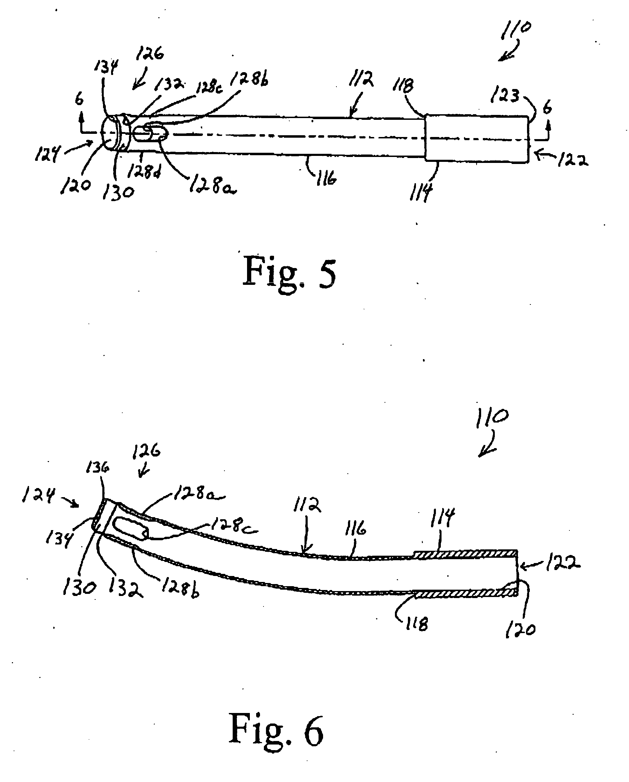

While the cannula 40 of Fig. 1 is axially straight (i.e. non-curved), a cannula made in accordance with aspects of the subject invention may be curved in various degrees. As such, referring now to Figs. 5 and 6, there is depicted a curved embodiment of a cannula generally designated 110. At the outset, it should be appreciated that the degree of curve (i.e. the amount of offset from axially straight) is only exemplary. As such, a cannula may be made in accordance with the subject invention that are curved less than the degree of curvature of the cannula 110, or may be greater. Additionally, the cannula may be of different lengths.

The cannula 110 is one of a set of instruments or devices for performing a surgical procedure on articular cartilage, the other instruments of the set being shown in the other figures and described herein. Again, while described more fully below, the surgical procedure and one or more of the various instruments of the present instrument set, guides the various instruments through an incision of the body, gauges the depth of articular cartilage at a cartilage defect or focal lesion (site) of a joint that is proximate the incision, prepares the site, and allows implanting of an ACRU at the site.

The cannula 110 is made from a suitable surgical material such as metal and, preferably, but not necessarily, from stainless steel. Other suitable surgical materials, however, can be used and are contemplated. The cannula 110 is defined by a cylindrical, tubular or similarly shaped body 112 having a first portion 114 and a second portion 116. A bore 120 extends the length of the body 112 through the first portion 114 and the second portion 116. The bore 120 defines a first opening 122 in a proximal end of the cannula 110 and a second opening 124 in a distal end of the cannula 110.

While the bore 120 has the same diameter throughout the first portion 114 and the second portion 116, the outer diameter of the first portion 114 is greater than the outer diameter of the second portion 116. This creates an annular ledge 118 defining a boundary between the outer diameter of the first portion 114 and the outer diameter of the second portion 116. The bore 120, and thus the cannula 110 itself, serves as a guide to pass and/or utilize the various instruments of the present instrument set (and other instruments not shown and/or described herein) through an incision in the body. While the incision and the procedure is preferably arthroscopic in nature, the various instruments of the present instrument set may be utilized in surgical procedures that are not arthroscopic in nature.

It should be appreciated that the bore 120 may be made in various diameters. As such, the outer diameter of the first and second portions 114, 116 may be fashioned in various diameters. A surgical set of cannula tubes of various outer diameters and/or various diameter bores may be provided. In this manner, a surgeon may have a choice of sizes of cannula.

In like manner to the cannula 40 of Figs. 1 and 2, the cannula 110 terminates at a distal end in a distal tip 126. The distal tip 126 is configured to be a cartilage cutter that is utilized to prepare and/or aid in the preparation of the articular cartilage site. The distal tip 126 includes one or more windows or openings 128. The distal tip 126 of the cannula 110 has four windows 128a, 128b (that is diametrically opposite window 128a), 128c and 128d (that is diametrically opposite window 128c). Placement and number of windows may vary as desired and/or appropriate. Additionally, the windows 128a-d are fashioned as ovals or ovoid shaped openings. The shape and/or size of the windows may vary.

The distal tip 126 also has an annular cutting section 130. The cutting section 130 is here formed as a reduced diameter portion and as such, defines an annular rim 132. At the end of the cutting section 130 is a blade 134. The blade 134 is annular and defined by a tapered, angled or bevelled edge 136. The blade 134 may be fashioned in another shape if desired. The blade 134 is adapted, configured, and/or operative to cut an area of cartilage (e.g. a focal lesion or damaged cartilage) from the articular cartilage site. The length of the blade 134 determines the maximum depth of articular cartilage cut. The incised area of cartilage corresponds to the shape of the blade 134 and thus corresponds, as well, to the size of the bore 120 at the distal bore end 124. The cannula 110 is both a guide for instruments via passing of an instrument through the bore 120 thereof, and an articular cartilage cutter through utilization of the distal tip 126. The cannula 110 is thus a dual purpose instrument.

In order for the obturator 70 (see Fig. 3) to be able to bend through the curvature of the bore 120 of the cannula 110 (and, of course, all curved cannula) the body 72 may be made of a bendable or resilient material. This would allow the obturator to appropriately extend through the bore 120, but return to its original shape. Alternatively, while not shown, an obturator may be made whose body has the same degree of curvature as the cannula in which it is to be used. Therefore, a set of obturators may be provided in the instrument set to correspond to a set of like-curved cannula in the instrument set.

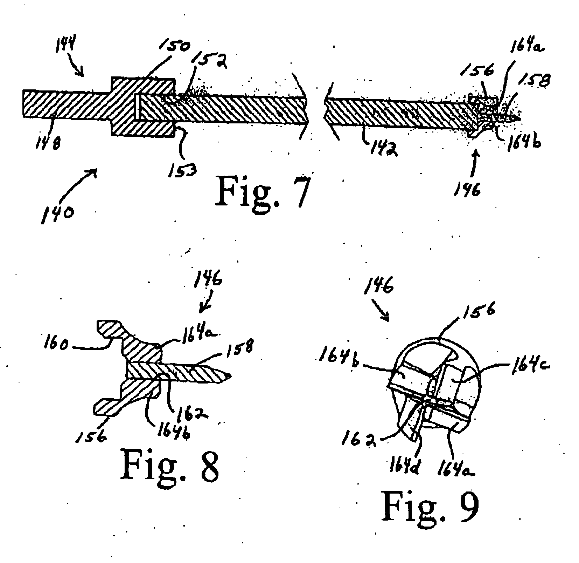

Because the cannula 110 is bent or curved, it is not possible for the rigid reamer 90 of Fig. 4 to extend through the bore 120. In view of this, the reamer must take another form. Referring now to Fig. 7, there is depicted a reamer, generally designated 140, that may be used with any curved cannula. This is because the reamer 140 is flexible. The nature of the flexibility of the reamer 140 will become evident with the below description.

The reamer 140 includes a flexible shaft 142 fabricated from a braided, segmented, coiled or similar construction material suitable for surgical use. The flexible shaft 142 is constructed such that the shaft 142 may bend to fit the curvature of the bore of the selected cannula, while still allowing rotation of the shaft to effect reaming/cutting by rotation of a reaming/cutting head 146 attached to a distal end of the shaft 142. The proximal end of the shaft 142 is non-rotatably connected to an attachment device 144 that is adapted, configured and/or operative to be attached to a rotation device (not shown). The attachment device 144 includes an attachment shaft 148 for receipt by the rotation device. A socket portion 150 extends from an end of the attachment shaft 148 and includes a bore 152 that receives an end of the shaft 142. The shaft 142 is non-rotatably received in the bore 152 such that as the attachment device 144 is rotated, the shaft 142 is also rotated.

The reamer 140 also includes a reaming, cutting and/or site preparation head or tip 146 that is provided on a distal end of the shaft 142. The reaming/cutting head 146 is non-rotatably attached to the end of the shaft 142 and is configured and/or operative to cut through cartilage (e.g. meniscus and articular) and to simultaneously bore a hole in subchondral bone. As seen in Fig. 8, the reaming/cutting head 146 is characterized by a body 156 having a shaft bore 160 that receives the shaft 142. Moreover, the reaming/cutting head 146 has a plurality of reaming/cutting blades 164, here embodied as four such blades, of which two of these blades 164a and 164b are seen in Fig. 7. Fig. 9 may be referenced for depicting all four blades 164a, 164b, 164c and 164d. Of course, it should be appreciated that while the reaming/cutting head 146 has four blades, the reaming/cutting head 146 may have more or less blades as well as other styles of blades.

Referring additionally to Figs. 8 and 9, the reaming/cutting head 146 has a boring tip 158 that axially extends from a bore 162 in the body 156. The boring tip 158 is adapted, configured and/or operative to bore a hole in the subchondral bone underneath the reamed articular cartilage. The size and length (dimensions) of the boring tip 158 is selected so as to bore a hole appropriate for receiving the stem of an articular repair device such as is known in the art. Thus, as the reamer 140 is rotated, the appropriate sized hole is bored into the subchondral bone while the blades 164 ream out the previously incised area of articular cartilage.

The reamer 140 (i.e. the various parts thereof) may be fashioned in various dimensions in order to be accommodated in a particular length of cannula and/or to effect appropriate cutting depths of both the bore in the subchondral bone for the shaft of the articular cartilage repair device and the cartilage at the cartilage repair site (having been previously cut via the cutting blade of the cutting head of the cannula). In all cases, however, the length of the elongated shaft 142 including the reaming/cutting head 146 (i.e. from a stop surface 153 defined on the socket head 150, see Fig. 7, to an end of the cutting blades 164) is sized such that when the stop surface 153 abuts the end 123 of the cannula 110 (see Fig. 5), at least a portion of the blades 164 extends beyond the end of the cutting portion 126 of the cannula 110. The amount of blade extension is sufficient to ream out the incised depth of the articular cartilage.

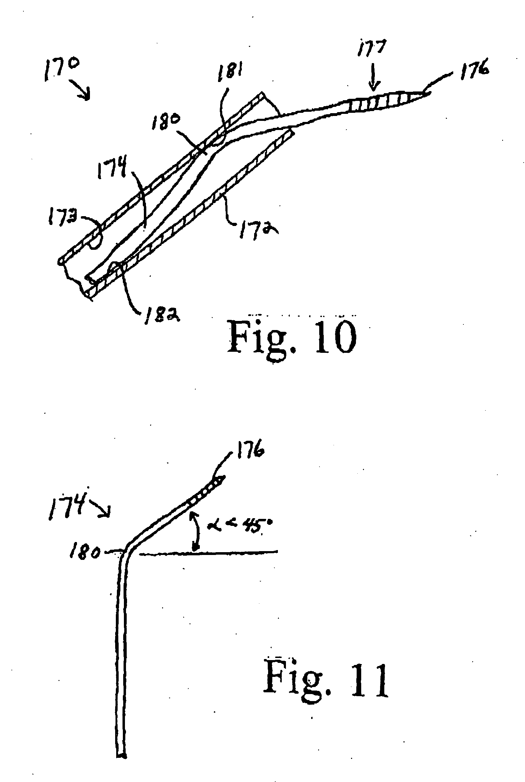

Referring now to Fig. 10, there is depicted an operative portion of an articular cartilage thickness gauge, generally designated 170 in accordance with an aspect of the subject invention. The articular cartilage thickness gauge 170 may be part of the instrument set for preparing and/or implanting an ACRU as described herein. Only an operative portion of the articular cartilage thickness gauge is depicted, since the other aspects of the thickness gauge may be fashioned in a number of known manners.

The articular cartilage thickness gauge 170 includes a tube 172 that is preferably, but not necessarily, made of a resilient material. The tube 172 has an inner bore 173 in which is disposed a needle, probe or the like 174. The probe 174 terminates in a tip 176 and includes a number of gauge marks 177. The gauge marks correspond to various depths measured from the end of the tip 176. Such gauge marks may be demarcated in millimetres or other small increments. An end of the probe 174 that is not seen in Fig. 10 is attached to a handle mechanism that allows the probe 174 to extend and retract from the tube 172. Thus, the probe 174 is slidably disposed in the bore 173 such that the tip 176 may retract into the tube 172 (i.e. its tip being within the bore 173) when not in use or during insertion through a particular cannula, and may extend beyond the end of the tube 172 during use.

Referring additionally to Fig. 11, the probe 174 is formed with a bend 180 proximate the tip 176. The bend 180 provides an angle α of preferably less than 45 ° as shown in Fig. 11. Then, when deployed, the probe 174 will have an angle α of approximately 45 °. When the probe 174 is disposed in the tube 172 as depicted in Fig. 10, the bend 180 causes the probe to have two contact points 181 and 182 with the inner wall of the bore 173. The contact point 181 is at or proximate the bend 180, while the contact point 182 is further down the probe. The contact points provide stability to the probe 174 when deployed and/or taking a depth measurement of the articular cartilage.

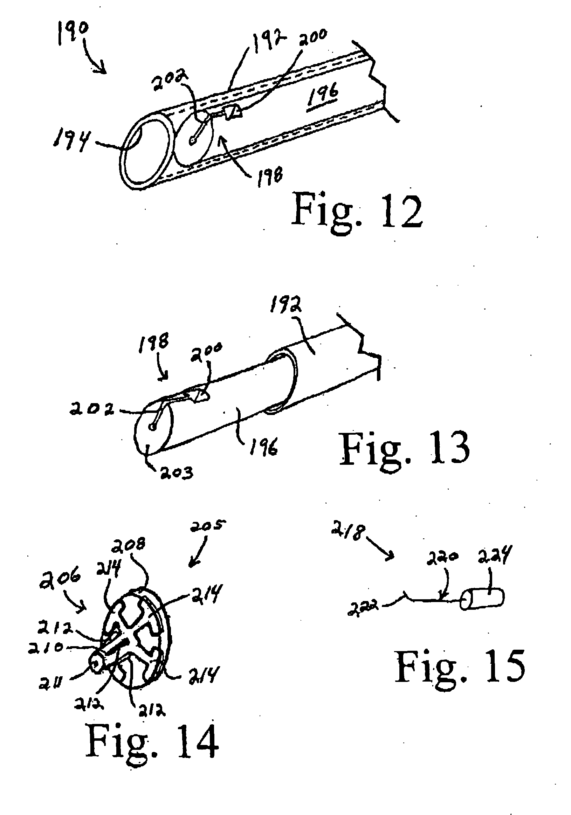

Referring now to Fig.12, there is depicted an embodiment of an implant delivery device generally designated 190. The implant delivery device 190 fashioned in accordance with an aspect of the subject invention. While the implant delivery device 190 is an instrument unto itself, the implant delivery device 190 is one of a set of instruments or devices for performing a surgical procedure on articular cartilage, the other instruments of the set being shown in the other figures and described herein.

The implant delivery device 190 is adapted, configured and/or operative to receive, hold, implant, and release an articular cartilage repair unit (ACRU) or device and articular cartilage matrix (collectively, ACRU device) such as that shown in Fig. 14 and described hereinbelow. The implant delivery device 190 includes an outer sleeve, tube, cylinder or the like 192 that defines an inner bore 194, the inner bore 194 having a given inner diameter. The sleeve 192 has an outer diameter that is sized to be receivable in the bore of a selected cannula as described herein. The implant delivery device 190 is therefore adapted, configured and/or operative to be received in and extend through the selected cannula.

The implant delivery device 190 further includes a pusher rod or cylinder 196 disposed within the bore 194 of the sleeve 192. The pusher rod 196 is axially slidable within the sleeve 192, the purpose of which will become evident with the below description. The pusher rod 196 includes a configured cutout 198 for receiving and holding an implant retainer (see Fig. 15, for example, and its accompanying description below) that itself retains an ACRU assembly for eventual implantation of the ACRU thereof. The configured cutout 198 includes a retainer slot 200 and wire slot 202 that extends from the retainer slot 200 to an end 203 of the pusher rod 196. The retainer slot 200 is configured in the same shape as a head of the implant retainer, while the wire slot 202 is shaped to receive a wire of the implant retainer.

The implant delivery device 190 is depicted in Fig. 13 in a pre-loaded (i.e. before an ACRU assembly and implant retainer has been inserted or received therein), but extended state ready to receive the ACRU assembly and implant retainer. The pusher rod 196 is thus extended from the sleeve 192 and ready to accept the ACRU assembly on the implant retainer.

Referring to Fig. 15, there is depicted an implant retainer, generally designated 218. The implant retainer 218 has a retainer or retainer portion 224 that holds a wire 220. The retainer 224 is cylindrical or tubular in shape. The wire 220 is preferably fabricated from nitinol or other suitable metal material that is bendable without breaking. The wire 220 includes a bend 222 distal from the retainer portion 224. The wire 220 thus forms a "J" and may therefore be known as a J-wire.

The retainer 218 may be formed of a suitable, surgical appropriate plastic in which case the J-wire 220 may be integrally molded therein, such as via insert molding. Alternatively, the retainer 218 may be formed of a suitable, surgical appropriate metal in which case the retainer 218 may be crimp-fit around the J-wire 220. Other materials and/or methods of attachment, however, may be used. The retainer 218 is shaped at least substantially the same as the retainer slot 200, while the wire slot 202 is configured to receive the J-wire 220.

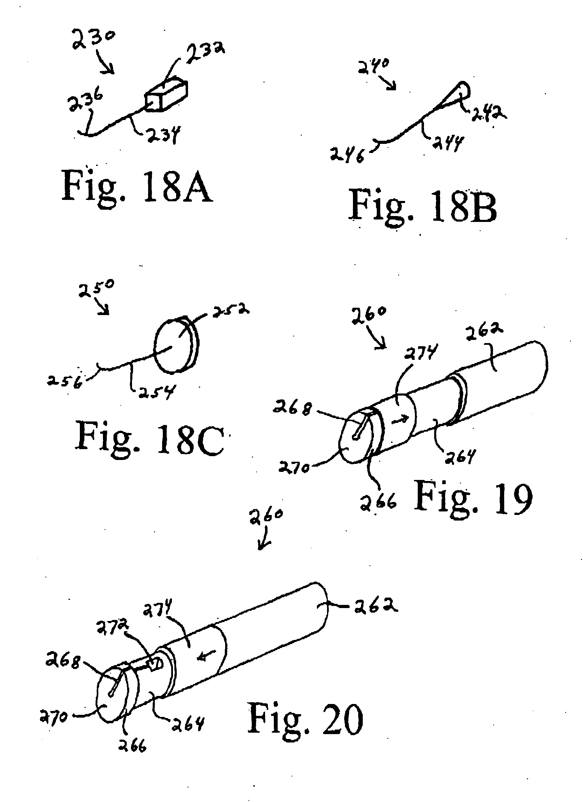

It should be appreciated that the retainer of the implant retainer may take different forms. Fig. 18A depicts an alternative implant retainer generally designated 230 that has a rectangular shaped retainer 232. A wire 234 having a bend 236, forming a J-wire, extends from the retainer 232. Fig. 18B depicts another alternative implant retainer generally designated 240 that has a conical shaped retainer 242. A wire 244 having a bend 246, forming a J-wire, extends from the retainer 242. Fig. 18C depicts yet another alternative implant retainer generally designated 250 that has a flat circular disk shaped retainer 252. A wire 254 having a bend 256, forming a J-wire, extends from the retainer 252. In all cases, of course, the retainer slot 200 would be configured accordingly to accommodate the configuration of the retainer.

Referring now to Fig. 14, there is depicted an ACRU assembly 205 that may be implanted into subchondral bone as provided herein by one of the present implant delivery devices. The ACRU assembly 205 includes an ACRU fixation device 206 with an articular cartilage matrix or the like 208 such as is known in the art. The ACRU fixation device 206 is preferably fabricated from a suitable surgical-appropriate polymer such as is known in the art. The ACRU fixation device 206 includes a stem 210 that axially extends from a plurality of spokes 214. Particularly and preferably, the stem 210 axially extends from a centre of the spokes 214. The stem 210 includes a plurality of fins, barbs or the like 212 that radially extend from the stem 210. A bore 211 is provided through the stem 210 and centre of the spokes 214. The bore 211 is sized to receive the J-wire of an implant retainer.

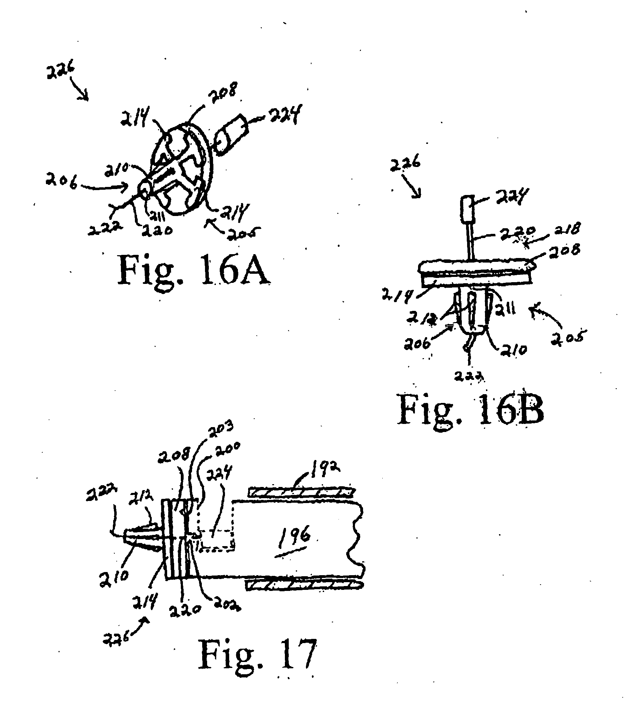

Figs. 16A and 16B depict an ACRU implant assembly 226 that includes an ACRU assembly 205 situated on the implant retainer 218. Particularly, the J-wire 220 extends through the bore 211 such that the bend 222 extends beyond the end of the stem 210. Therefore, the length of the wire 220 from the retainer 224 to the bend 222 is such that it allows the receipt of the ACRU fixation device 206 thereon, with the bend 222 proximate but beyond the end of the stem 210, and the length of the exposed wire between the retainer 224 and the top of the matrix 208 sufficient to span the length of the area between the end 203 of the pusher rod 196 and the retainer slot 200.

In Fig. 17, the ACRU implant assembly 226 is shown captured or retained in the pusher rod 196. The ACRU assembly is now ready to be implanted by the implant delivery device through the particular cannula. The retainer 224 is situated in the retainer slot 200, while the J-wire 220 is situated in the wire slot 202. The articular cartilage matrix 208 is adjacent (preferably abutting) the end 203 of the pusher rod 196. The sleeve 192 is shown in a retracted position as it would be when inserting the ACRU implant assembly thereon. Thereafter, the sleeve 192 would cover the ACRU assembly for implanting the ACRU assembly through the selected cannula.

Referring now to Figs. 19 and 20, there is depicted another embodiment of an implant delivery device, generally designated 260, in accordance with an aspect of the subject invention. The implant delivery device 260 provides a sliding sleeve lock mechanism for receiving and retaining an ACRU implant assembly. The implant delivery device 260 includes a handle 262 that is sized to be received in the bore of the selected cannula, A reduced diameter shaft 264 axially extends from the handle 262 and terminates in a seating ring 266. The seating ring 266 is of a larger diameter than the shaft 264. The seating ring 266 defines a seating surface 270 on an end face thereof.

The shaft 264 includes a retainer slot 272 configured to receive one of the retainers of the implant retainers described herein. A wire slot 268 extends from the retainer slot 272 to the end 270. A sliding sleeve 274 is disposed around the shaft 264 between the seating ring 266 and the handle 262. The sliding sleeve 274 is biased such as by spring loading to be in a closed position as depicted in Fig. 19. Movement of the sleeve as depicted by the arrow in Fig. 19 exposes the retainer slot 272 and a portion of the wire slot 268 as depicted in Fig. 20. The shaft 264 from the retainer slot 272 to the handle 262 is sized to accommodate the length of the sleeve 274 in order to fully exposed the retainer slot 272. The implant delivery device is now ready to receive an ACRU implant assembly such as the ACRU implant assembly 226 depicted in Figs 16A and 16B.

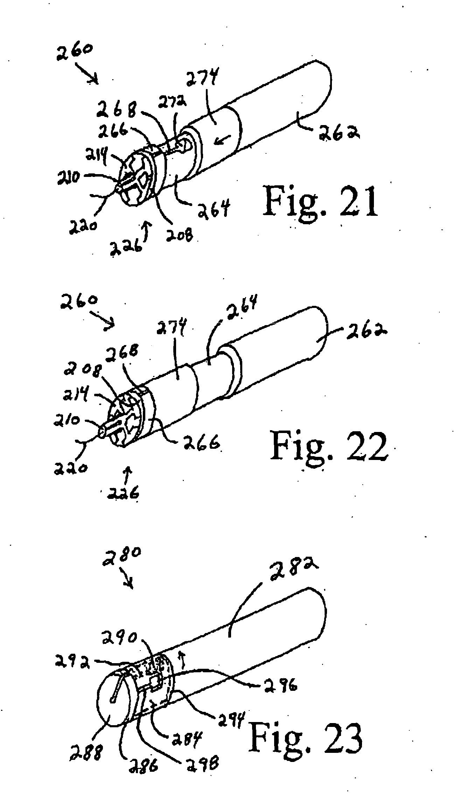

Fig. 21 depicts the ACRU implant assembly 226 inserted in and retained by the implant delivery device 260. It should be appreciated that the sleeve 274, being biased to be in a closed position as depicted in Fig. 19 (and indicated by the arrow in Fig. 21), must be held against such bias by an operator (not shown). After inserting the ACRU implant assembly 226 into the implant delivery device 260 and the release of the sleeve 274, the sleeve 274 is biased against the seating ring 266 as depicted in Fig. 22. The sleeve 274 thus covers the retainer slot 272 and a portion of the wire slot 268. The ACRU assembly is now ready to be implanted through the cannula.

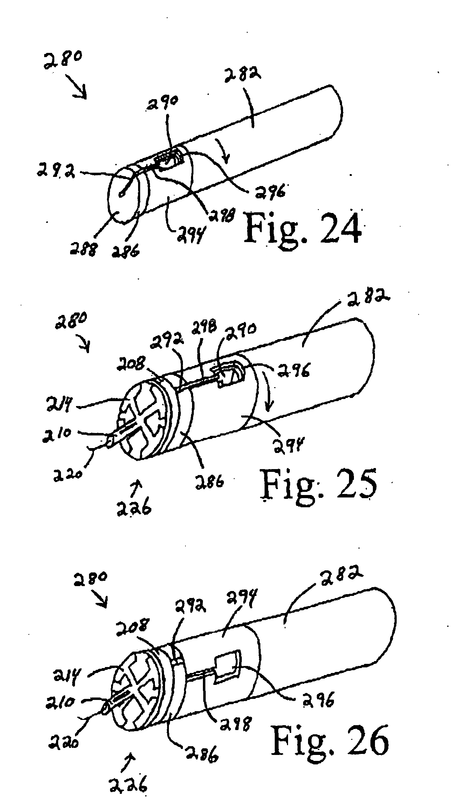

Referring now to Figs. 23 and 24, there is depicted yet another embodiment of an implant delivery device generally designated 280. The implant delivery device 280 may be one instrument of the set of instruments. The implant delivery device 280 has a handle 282 that is sized to fit into the bore of the selected cannula. The handle 282 includes a reduced diameter shaft portion 284 (shown in phantom) on an axial end thereof. The shaft portion 284 includes a seating ring 286 on an axial end thereof that defines an end or face 288 thereof. The shaft portion 284 includes a retainer slot 290 (shown in phantom) and a wire slot 292 (shown in partial phantom) that extends from the retainer slot 290 though the end 288 of the seating ring 286. The retainer slot 290 is configured the same as the retainer of the particular implant retainer, while the wire slot 292 is sized to receive the J-wire of the implant retainer.

A cylindrical sleeve 294 is disposed around the shaft portion 284 and includes a retainer port 296 and a wire channel 298 that extends from the retainer port 296. The cylindrical sleeve 294 is limitedly rotatable about the shaft portion 284. In Fig. 23, the sleeve 294 is in a closed position, but is rotatable as indicated by the arrow. In Fig. 24, the sleeve 294 is in an open position, but is rotatable as indicated by the arrow. The sleeve 294 is thus rotatable between the open and closed positions. In the closed position as depicted in Fig 23, the retainer port 296 of the sleeve 294 does not register (align) with the retainer slot 290 of the shaft portion 284. Additionally, the wire channel 298 of the sleeve 294 does not register (align) with the wire slot 292 of the shaft portion 284. In the open position as depicted in Fig. 24, the retainer port 296 of the sleeve 294 registers (aligns) with the retainer slot 290 of the shaft portion 284. Additionally, the wire channel 298 of the sleeve 294 registers (aligns) with the wire slot 292 of the shaft portion 284.

Preferably, but not necessarily, the sleeve 294 is biased into the closed position by a spring mechanism or the like. Therefore, in order to position the sleeve 294 into the open position as depicted in Fig. 24, the bias may be overcome manually. The open position as depicted in Fig. 24 allows the insertion of the ACRU implant assembly 226.

Referring to Fig. 25, the sleeve 294 is shown in the open position. The ACRU implant assembly 226 has been inserted into and thus has been received by the implant delivery device 280. In Fig. 26, the sleeve 294 has rotated into the closed position (as illustrated by the arrow in Fig. 25). The ACRU implant assembly 226 is not able to be removed without rotating the sleeve 294 back into the open position. The ACRU assembly is ready to be implanted. Again, this is preferably, but not necessarily, accomplished through the cannula of the present invention.

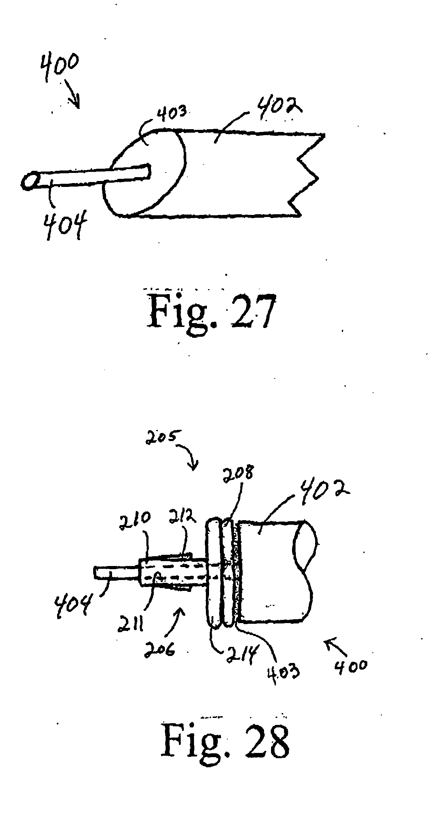

Referring now to Fig. 27, there is depicted another alternative embodiment of an implant delivery device generally designated 400. The implant delivery device 400 includes a handle, tube, body or the like 402 that is dimensioned to extend through a cannula as herein provided. An end 403 of the body 402 has an ACRU implant retainer 404 that is operative, configured and/or adapted to releasably retain an ACRU assembly. In the embodiment of the implant retainer 404 of Fig. 27, the implant retainer 404 is formed by a wire. The ACRU implant retainer 404 may be a nitinol type wire such as discussed above.

The implant retainer 404 is sized to receive and releasably retain an ACRU. Fig. 28 provides a side view of the implant delivery device 400 with an ACRU 205 retained thereon. Particularly, the implant retainer 404 extends through the bore 211 of the ACRU to releasably retain the ACRU. The matrix 208 is adjacent the end 403 of the body 402 when the ACRU is appropriately situated on the implant retainer 404.

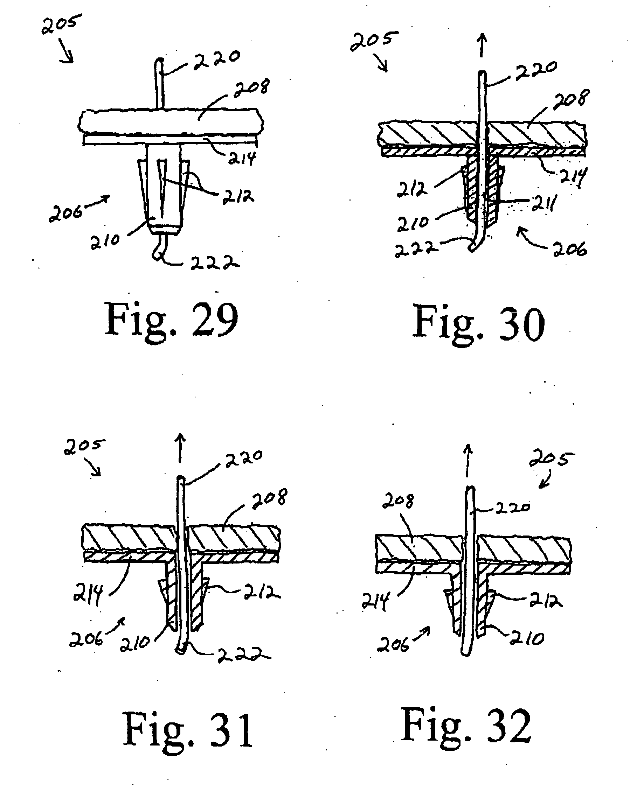

Figs. 29-32 illustrate how the ACRU assembly 205 (the fixation device 206 and the matrix 208) is implanted or released from any one of the implant retainers described herein. Only the ACRU assembly 205 is shown, not the bone or surrounding articular cartilage. In Fig. 29, the ACRU assembly 205 is shown in an initially implanted state. In Fig. 30, as the implant delivery device begins to pull (as represented by the upward pointing arrow) the wire 220 from the ACRU 206 (by virtue of the retainer portion of the implant retainer that is attached to the wire 220) the bend 222 of the wire 220 begins to straighten. In Fig. 31, upon further extraction of the wire 220 as represented by the arrow above the wire, the bend or hook 222 continues to straighten. Finally, as illustrated in Fig. 32, continued extraction of the wire 220 from the ACRU fixation device 206 by the implant delivery device completely straightens out the bend (and therefore not seen in Fig. 32) so that the wire 220 freely exits from the ACRU fixation device 206. Thereafter, the implant delivery device may be removed from the cannula.

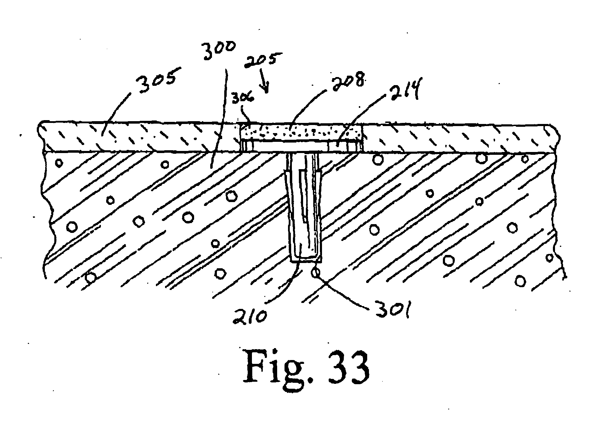

Referring now to Fig. 33, there is depicted a representation of the ACRU assembly 205 implanted into subchondral bone 300, the matrix 208 being aligned with the natural articular cartilage 305. Particularly, the stem 210 is implanted into a bore 301 formed in the subchondral bone 300 by the tip of the reamer, while the matrix 208 and support structure 214 rests in a reamed out portion 306 of the articular cartilage 305 by the reamer blades.

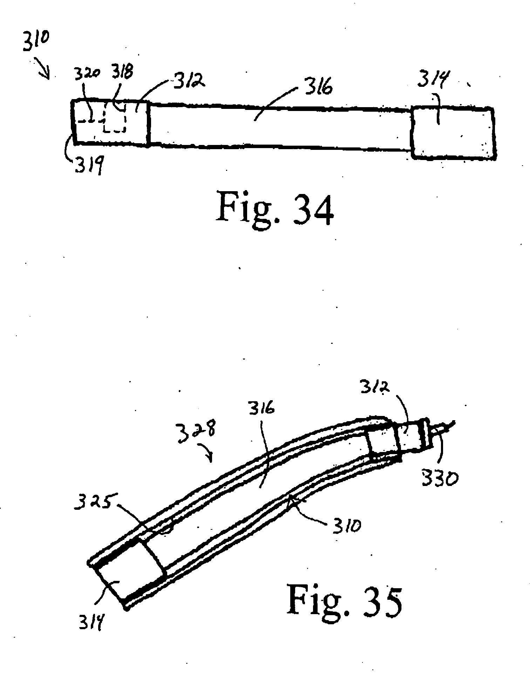

It should be appreciated that the various implant delivery devices as described above are usable only with the axially straight (non-curved) cannula. This is due to the rigid nature of the components. In order to alleviate this problem and thus be able to utilize any one of the various implant delivery devices, any of the above-described implant delivery devices may be formed as shown in Fig. 34. In Fig. 34, there is depicted an elementary embodiment of a flexible implant delivery device generally designated 310.

The flexible implant delivery device 310 has a distal tip 312 that is made of a hard, surgical-appropriate material. The tip 312 includes a retainer cutout or slot 318 that is configured to receive a retainer portion of an implant retainer. A wire slot 320 extends from the retainer cutout 318 to the end 319 of the tip 312. The flexible implant delivery device 310 also includes a proximal end 314 that is fabricated of a hard, surgical-appropriate material. A shaft 316 is disposed between the tip 312 and the end 314. The shaft 316 is fabricated from a flexible, surgical-appropriate material. The flexible shaft 316 is thus operative to bend or curve in any direction and/or amount in order to be accommodated in a cannula of any curvature. The flexible shaft 316 is also resilient such that the shaft will, without external bias, tend to remain in (or go back into) a straight configuration.

The ability to flex or bend is illustrated in Fig. 35. Fig. 35 depicts a curved cannula 328 having a curved bore 325. The flexible implant delivery device 310 is shown disposed in the bore 325 with the flexible shaft 316 bent to fit the curvature of the bore 325. The distal tip 312 is shown retaining an ACRU assembly 330 as provided herein. The handle (proximate end 314) extends out from the cannula 318 for use in inserting the ACRU assembly 330 into the prepared articular cartilage site and for implanting the ACRU assembly 330 in the subchondral bone underneath the prepared articular cartilage.

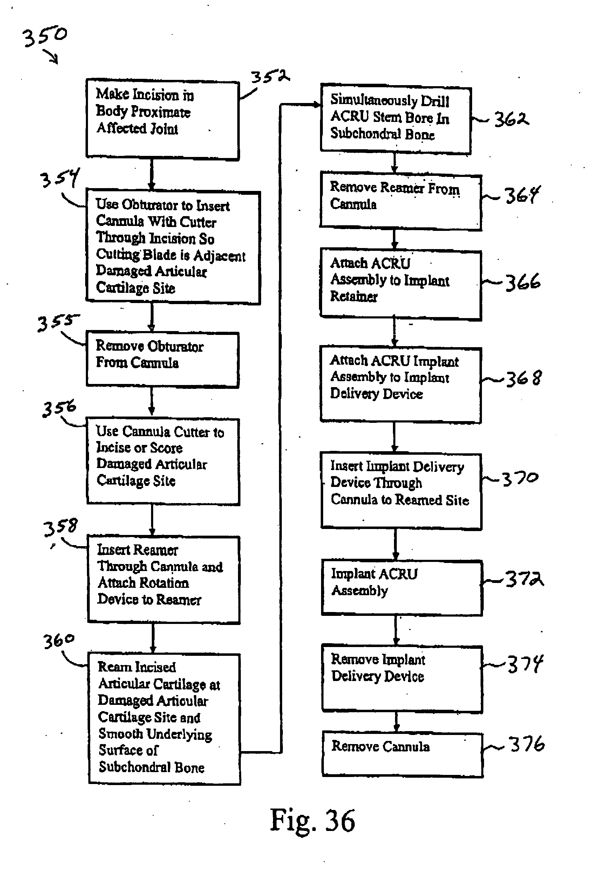

A method or technique of preparation of an articular cartilage site and implantation of an articular cartilage repair unit/device or ACRU in accordance with an aspect of the subject invention will now be described utilizing a set of instruments as described herein that includes at least some, if not all of the present instruments. As well, reference will be made to Fig. 36. Fig. 36 depicts a flowchart, generally designated 350, of the method or technique. It should initially be appreciated that the blocks or steps of the method 350, relate to a technique for repairing a damaged articular cartilage site (damaged articular cartilage, e.g. focal lesion). This technique includes the use of the tools (implements or components) described herein.

In block or step 352, an incision or incisions is first made in the body of a patient proximate to the affected joint in a manner known in the art. The incision(s) may be for an arthroscopic procedure or not. Once the incision(s) has been made, in block 354, an obturator is used to insert a selected cannula in accordance with the subject invention through the incision (or one of the incisions). The selected cannula may be a straight cannula or may be a curved cannula. If the selected cannula is a curved cannula, the surgeon must select the degree of curvature. Additionally, the size (diameter) of the cannula must be selected. Therefore, the instrument set should include a plurality of cannula from straight to variously curved cannula each coming in various sizes.

In block 354, the selected cannula is inserted through the incision using the obturator and situated adjacent (i.e. over) the affected or damaged articular cartilage site. This positions the cutting tip/blade of the cannula around the damaged articular cartilage site. The obturator is then removed from the cannula (block 355).

In block 356, once the cannula is in a proper position over the damaged articular cartilage site, the damaged articular cartilage is cut or incised by the cannula blade. The cartilage is incised to the proper depth. The depth of cutting may first be ascertained by use of the articular cartilage depth gauge instrument that may be part of the instrument set.

Thereafter, in block 358, an appropriate reamer is selected and inserted into the cannula. The reamer is attached to a rotation device for rotation of the reamer. In block 360, the articular cartilage incised by the cannula is then reamed by the blades of the reamer. The reamer also reams the subchondral bone under the articular cartilage flat or smooth. Simultaneously, in block 362, a bore is drilled in the subchondral bone for receipt by the stem of the ACRU fixation device. In block 364, the reamer is then removed from the cannula. The site is now ready to receive the ACRU implant.

In block 366, an ACRU assembly is attached to an implant retainer. Thereafter, in block 368, the ACRU implant assembly is attached to the implant delivery device. In block 370, the implant delivery device is then inserted through the cannula to the prepared site. The ACRU assembly, in block 372, is then implanted into the prepared site. In block 374, the implant delivery device is then removed from the cannula. Thereafter, the cannula 376 is removed from the incision.

## Claims

1. A set of surgical tools for preparing a damaged articular cartilage site on subchondral bone of a joint and implanting an articular cartilage repair device comprising:
a cannula (40) comprising:
a tubular body (42) defining a proximal and a distal end, with a bore (50) disposed in said tubular body and extending from said proximal end to said distal end; and
an annular blade which is provided by the tip (56) of said distal end of said tubular body, for incising an area of articular cartilage;
a surgical drill (90) comprising:
a drill shaft (92) having a proximal end and a distal end;
an attachment tip on said proximal end of said drill shaft which can be fitted into a device which can rotate the shaft; and
a site preparation tip (100) on said distal end of said drill shaft for reaming the area of the damaged articular cartilage which has been incised by the annular blade and simultaneously for cutting a bore in the subchondral bone underneath the reamed area of the damaged articular cartilage; and
an implant delivery device (260) comprising:
a handle (262) defining a proximal end and a distal end;
a shaft (264) extending from said distal end of said handle and having an application end with a retention slot (272) configured to releasably receive an articular cartilage repair assembly comprising an articular cartilage repair unit releasably retained on an articular cartilage implant retainer; and
a retaining sleeve (274) disposed on said shaft and operative in a first mode to allow the articular cartilage implant retainer to be received in said retention slot, and in a second mode that prevents egress of the articular cartilage implant retainer from said retention slot,
in which the tubular body of the cannula is sized so that (a) the surgical drill can fit into the bore of the body allowing the articular cartilage site and underlying bone to be prepared through the cannula, and (b) the implant delivery device can fit into the bore of the cannula body allowing the articular cartilage repair unit to be implanted at the articular cartilage site through the cannula.

2. The set of surgical instruments of claim 1, further comprising at least one and up to all of:
a plurality of cannulae (40) having various cannula dimensions;
a plurality of surgical drills (90) having various drill dimensions; and
a plurality of implant delivery devices (260) having various implant delivery characteristics.

3. A set of instruments as claimed in claim 2, wherein said plurality of surgical drills (90) each has either or both of a rigid shaft and a flexible shaft.

4. A set of instruments as claimed in claim 2, wherein said various cannula dimensions comprise outer diameter of the tubular body, bore diameter, and tubular body length.

5. A set of instruments as claimed in claim 2, wherein said implant delivery characteristics include retention slot configuration and retention mechanism.

6. A set of instruments as claimed in claim 1, further comprising an articular cartilage thickness gauge dimensioned to extend through said cannula and operative to measure a thickness of articular cartilage at the damaged articular cartilage site.

## Patentansprüche

1. Ein Satz chirurgischer Werkzeuge zum Präparieren eines beschädigten Gelenkknorpelbereichs auf einem subch,ondralen Knochen eines Gelenks und Implantieren einer Gelenkknoipelreparaturvorrichtung, umfassend:
eine Kanüle (40), umfassend:
einen röhrenförmigen Körper (42), der ein proximales und ein distales Ende definiert, wobei eine Bohrung (50) in dem röhrenförmigen Körper angeordnet ist und sich von dem proximalen Ende zu dem distalen Ende erstreckt; und
eine ringförmige Klinge, die durch die Spitze (56) des distalen Endes des röhrenförmige Körpers bereitgestellt wird, zum Einschneiden eines Gelenkknorpelgebiets;
einen chirurgischen Bohrer (90), umfassend:
einen Bohrerschaft (92), der ein proximales Ende und ein distales Ende aufweist;
eine Befestigungsspitze an dem proximalen Ende des Bohrerschafts, die in eine Vorrichtung eingepasst werden kann, welche den Schaft drehen kann; und
eine Bereichspräparierungsspitze (100) an dem distalen Ende des Bohrerschafts zum Erweitern des Gebiets des beschädigten Gelenkknorpels, das durch die ringförmige Klinge eingeschnitten wurde, und gleichzeitig zum Schneiden einer Bohrung in den subchondralen Knochen unterhalb des erweiterten Gebiets des beschädigten Gelenkknorpels; und
eine Implantateinsetzvorrichtung (260), umfassend:
einen Handgriff (262), der ein proximales Ende und ein distales Ende definiert;
einen Schaft (264), der sich von dem distalen Ende des Handgriffs erstreckt und ein Anwendungsende mit einem Aufnahmeschlitz (272) aufweist, der konfiguriert ist, um eine Knorpelgewebereparaturanordnung lösbar aufzunehmen, die eine Knorpelgewebereparatureinheit umfasst, die an einer Gelenkknorpelimplantathalterung lösbar festgehalten wird; und
eine Haltehülse (274), die auf dem Schaft angeordnet ist und in einem ersten Modus, um es der Gelenkknorpelimplantathalterung zu ermöglichen, in dem Aufnahmeschlitz aufgenommen zu werden, sowie in einem zweiten Modus betreibbar ist, der ein Ablösen der Gelenkknorpelimplantathalterung von dem Aufnaluneschlitz verhindert,
wobei der röhrenförmige Körper der Kanüle so bemessen ist, dass (a) der chirurgische Bohrer in die Bohrung des Körpers passen kann, wodurch ermöglicht wird, dass der Gelenkknorpelbereich und darunterliegenden Knochen durch die Kanüle präpariert werden, und (b) die Implantateinsetzvorrichtung in die Bohrung des Kanülenkörpers passen kann, wodurch ermöglicht wird, dass die Gelenkknorpeleinheit durch die Kanüle an dem Gelenkknorpelbereich implantiert wird.

2. Satz chirurgischer Instrumente nach Anspruch 1, ferner umfassend wenigstens eine und bis hin zu allen der folgenden:
eine Vielzahl von Kanülen (40), die verschiedene Kanülenabmessungen aufweisen;
eine Vielzahl chirurgischer Bohrer (90), die verschiedene Bohrerabmessungen aufweisen; und
eine Vielzahl von Implantateinsetzvorrichtungen (260), die verschiedene Implantateinsetzoharakteristiken aufweisen.

3. Satz von Instrumenten nach Anspruch 2, **dadurch gekennzeichnet, dass** die Vielzahl chirurgischer Bohrer (90) jeweils entweder einen starren Schaft oder einen flexiblen Schaft oder beides aufweist.

4. Satz von Instrumenten nach Anspruch 2, **dadurch gekennzeichnet, dass** besagte verschiedene Kanülenabmessungen einen äußeren Durchmesser des röhrenförmigen Körpers, Bohrungsdurchmessers und Länge des rohrförmigen Körpers aufweisen.

5. Satz von Instrumenten nach Anspruch 2, **dadurch gekennzeichnet, dass** die Implantateinsetzcharakteristikcn eine Aufnahmeschlitzkonfiguration und einen Haltemechanismus enthalten.

6. Satz von Instrumenten nach Anspruch 1, ferner umfassend eine Gelenkknorpeldickenlehre, die dimensioniert ist, um sich durch die Kanüle zu erstrecken, und betreibbar ist, um eine Dicke des Gelenkknorpels an dem beschädigten Gelernkknoxpelbereich zu messen.

## Revendications

1. Ensemble d'instruments chirurgicaux pour préparer un site de cartilage articulaire endommagé sur l'os sous-chondral d'une articulation et implanter un dispositif de réparation de cartilage articulaire, comprenant :
✔ une canule (40) comprenant :
✔ un corps tubulaire (42) définissant une extrémité proximale et une extrémité distale, avec un alésage (50) disposé dans ledit corps tubulaire et s'étendant à partir de ladite extrémité proximale jusqu'à ladite extrémité distale ; et
✔ une lame annulaire qui est fournie par la pointe (56) de ladite extrémité distale dudit corps tubulaire, pour inciser une zone du cartilage articulaire ;
✔ un foret chirurgical (90) comprenant :
✔ une tige de foret (92) ayant une extrémité proximale et une extrémité distale ;
✔ un embout de fixation sur ladite extrémité proximale de ladite tige de foret qui peut être fixé dans un dispositif qui peut faire tourner la tige ; et
✔ un embout de préparation de site (100) sur ladite extrémité distale de ladite tige de foret pour aléser la zone du cartilage articulaire endommagé qui a été incisée par la lame annulaire, et pour couper simultanément un alésage dans l'os sous-chondral au-dessous de la zone alésée du cartilage articulaire endommagé ; et
✔ un dispositif de pose d'implant (260) comprenant .
✔ une poignée (262) définissant une extrémité proximale et une extrémité distale ;
✔ une tige (264) s'étendant à partir de ladite extrémité distale de ladite poignée et ayant une extrémité d'application avec une fente de retenue (272) configurée pour recevoir de manière amovible un ensemble de réparation de cartilage articulaire comprenant une unité de réparation de cartilage articulaire retenue de manière amovible sur un dispositif de retenue d'implant de cartilage articulaire ; et
✔ un manchon de retenue (274) disposé sur ladite tige et opérationnel dans un premier mode pour permettre au dispositif de retenue d'implant de cartilage articulaire d'être reçu dans ladite fente de retenue, et dans un deuxième mode qui empêche le dispositif de retenue d'implant de cartilage articulaire de sortir de ladite fente de retenue,
dans lequel le corps tubulaire de la canule est dimensionné de sorte que (a) le foret chirurgical peut s'adapter dans l'alésage du corps permettant au site de cartilage articulaire et à l'os sous-jacent d'être préparés par le biais de la canule, et (b) le dispositif de pose d'implant peut s'adapter dans l'alésage du corps de canule permettant à l'unité de réparation de cartilage articulaire d'être implantée sur le site de cartilage articulaire par le biais de la canule.

2. Ensemble d'instruments chirurgicaux selon la revendication 1, comprenant en outre au moins un et jusqu'à tous parme :
✔ une pluralité de canules (40) ayant différentes dimensions de canule ;
✔ une pluralité de forets chirurgicaux (90) ayant différentes dimensions de foret ; et
✔ une pluralité de dispositifs de pose d'implant (260) ayant différentes caractéristiques de pose d'implant.

3. Ensemble d'instruments selon la revendication 2, dans lequel ladite pluralité de forets chirurgicaux (90) ont chacun une tige rigide et/ou une tige flexible.

4. Ensemble d'instruments selon la revendication 2, dans lequel lesdites différentes dimensions de canule comprennent le diamètre externe du corps tubulaire, le diamètre de l'alésage, et la longueur du corps tubulaire.

5. Ensemble d'instruments selon la revendication 2, dans lequel lesdites caractéristiques de pose d'implant comprennent une configuration de fente de retenue et un mécanisme de retenue.

6. Ensemble d'instruments selon la revendication 1, comprenant en outre une jauge d'épaisseur de cartilage articulaire dimensionnée pour s'étendre à travers ladite canule et opérationnelle pour mesurer une épaisseur du cartilage articulaire sur le site de cartilage articulaire endommagé.
